# EUROPEAN PATENT SPECIFICATION

(11) **EP 1 028 316 B1**
(45) Date of publication and mention of the grant of the patent: **15.05.2002**
(21) Application number: 00500017.9
(22) Date of filing: 10.02.2000
(51) Int. Cl.: G01N 33/53

(54) **Device for carrying out agglutination tests**
Vorrichtung zur Durchführung von Agglutinierungstests
Dispositif pour l'exécution d'essais d'agglutination

(30) Priority: 12.02.1999 ES 9900372 U
(43) Date of publication of application: 16.08.2000
(73) Proprietor: Grifols Lucas, Victor, E-08150 Parets del Valles (Barcelona) (ES)
(72) Inventor: Grifols Lucas, Victor, E-08150 Parets del Valles (Barcelona) (ES)
(74) Representative: Duran Moya, Luis-Alfonso

(56) References cited:
- EP-A- 0 019 940
- EP-A- 0 820 812
- PATENT ABSTRACTS OF JAPAN vol. 015, no. 363 (P-1251), 12 September 1991 (1991-09-12) & JP 03 140867 A (OLYMPUS OPTICAL CO LTD), 14 June 1991 (1991-06-14)

## Description

The present invention provides a novel device having advantageous features which allows the performance of tests which include agglutination reactions necessary for carrying out various types of laboratory tests, for medical use, and, in particular, haematological tests and tests concerned with transfusion.

In particular, the tests are intended to detect an antigen/antibody reaction by agglutination.

As is known, immunohaematology is the discipline concerned with the determination of antigens located at the surface of the red corpuscles (which determine the blood groups), their interdependence, their relationship with any antibodies in the blood of other individuals and the relationship of their own antibodies towards red corpuscles of others, the antigenicity of the various antigens and the presence of abnormal antibodies. All this is aimed at the selection of the most suitable blood for a possible transfusion in order to avoid an immediate reaction and/or long-term sensitisation. The study of abnormal antibodies in pregnant women is also very important because sensitisation of the mother could lead to foetal erythroblastosis, which may have serious consequences for the foetus but, by meticulous diagnosis, bringing forward childbirth, ex-sanguineous transfusion etc., the prognosis can be greatly improved.

The above is intended to indicate the danger which may be posed by an error which, although difficult to commit in techniques in which only complete antibodies are used, as in the case of the basic groups O, A, B and AB, is easier to commit in the case of incomplete antibodies (also known as blocking antibodies), for which it is often necessary to use the Coombs test.

The basic blood groups (O, A, B and AB) were discovered by Lansteiner on observing that, when the red corpuscles of each of his co-workers were exposed to all of the sera of all of the others, agglutination occurred in some cases and not in others. After studying the combinations, he reached the conclusion that there were four blood groups (O, A, B and AB), and believed for the moment that the problem of transfusions had been solved. As regards those blood groups, the antigens, A and B, are always accompanied by the antibodies that are compatible with them (those of group A have the anti B antibody, those of group B have anti A and those of group O (O is not an antigen) have the anti A and anti B antibodies.

Those antibodies, which, like all antibodies, are gamma globulins, are of a molecular size called "19S", and it is owing to this large size that they agglutinate directly. They are therefore also called complete antibodies.

In the 1930s, other blood group systems were discovered, such as, for example, the P and MN systems, which differed from the ABO system in that the presence of compatible antibodies was not obligatory. It was also discovered that heredity followed Mendelian laws.

Despite the great advance made by the discovery of the ABO groups, owing, on the one hand, to the increase in the number of transfusions and, on the other hand, to the greater knowledge of immunohaematology, the conclusion was reached that some irregularities, such as foetal erythroblastosis or some transfusion reactions (especially in people who have undergone multiple transfusions) could be due to sensitisation caused by pregnancy or transfusions, by previously unknown antigens. Again, Lansteiner, this time together with Wiener, discovered the Rh factor (the name resulting from the similarity of the red corpuscles of a "Macacus Rhesus" monkey to an antigen).

The Rh system is composed of five antigens plus one position on a chromosome, which is inherited, but for which an antigen has hitherto not been found.

The new techniques which were developed as a result of the discovery of Rh gave rise to the discovery of more blood group systems and since all of them were inherited independently according to Mendelian laws, they were used, before the DNA test, for paternity exclusion tests.

The transfusion reactions and irregularities which may occur as a result of Rh and the other blood group systems are generally due to incomplete antibodies, gamma globulins of a "7s" size, which are not sufficiently long to act as a bridge between two antigen particles corresponding to two vicinal red corpuscles, the red corpuscles being somewhat spaced apart owing to their electrical charges, which is why agglutination does not occur in vitro.

Various techniques have been developed in order to detect the antigen antibody reaction by agglutination. Enzyme techniques and the concentrated albumin technique are based on a modification of the surface of the red corpuscles so that the antigen points are displayed and thus the small * 7 S * antibodies manage to act as a bridge between antigen points of different red corpuscles and thus bring about agglutination.

Nevertheless, despite the fact that its performance requires much more delicacy and skill, the test which is the most highly regarded is the Coombs test, which was developed by Coombs and Race and is also known as the anti-globulin test.

As is known, antibodies are gamma globulins, and in our case they are of human origin. When red corpuscles are immersed in a serum containing antibodies specific to their own antigens, the antibodies are fixed to the corresponding antigens of the red corpuscles but agglutination does not take place because the antibodies are not sufficiently long. Another reagent known as Coombs serum, anti-globulin serum or also antihuman serum now goes into action.

If a rabbit (or alternatively a goat, horse, etc.) is injected with human serum or human gamma globulin, the serum or gamma globulin acts as an antigen towards rabbits and the rabbit's immune system generates antihuman antibodies (antihuman protein) which exhibit affinity and attach (at both ends) to the antibodies which were previously fixed to the red corpuscles, thus bringing about agglutination.

The above, which thus explained seems so simple, harbours the problem that red corpuscles under investigation are immersed in human serum and consequently, if they were thus brought into contact with the anti-globulin serum, the latter would be rapidly neutralised by the human protein and agglutination would not take place. In order for the reaction to take place, the red corpuscles have to be washed three times by suspension with saline solution and centrifugation. That operation is not easy and is also time-consuming and therefore a large number of errors have occurred.

In the specific case of agglutination reactions which are carried out in small-diameter tubes containing the corresponding reactive serum and micro-beads and which, after centrifugation, make the reading of agglutination much easier, even permitting a certain degree of quantification, it is important to prevent the sample from coming into contact with the reactive serum and the micro-beads since the reading may be invalidated, especially in the specific case of the Coombs test, since the human protein which accompanies the sample red corpuscles may neutralise the mentioned Coombs serum because the human anti-globulin thereof is saturated by the human protein of the mentioned serum.

By working carefully, it is easy to avoid the mentioned contact because the surface tension of the reagent and of the sample facilitates the creation of an air bubble which has been found to be a good boundary. The work carried out with an automatic instrument such as the Diana performs the operations in such a manner that contact does not take place. The problem arises when the operation is carried out manually by a person who is not very competent. Contact between the sample and the anti-globulin serum, even before incubation, may reduce the strength of the serum and in deficient cases may lead to error.

Errors in immunohaematology may be not only serious but also fatal. The solution we propose increases the level of GLP (Good Laboratory Practices) in immunohaematology laboratories which use these methods.

The object of the present invention is to achieve much greater safety in devices used to carry out agglutination tests in immunohaematology.

In order to achieve its object, the present invention is based on the provision of a micro-test tube for agglutination which, by very simple means, prevents the sample to be analysed from being mixed with the test reagents before the incubation and centrifugation stages. For that purpose, the device to which the present invention relates comprises a body of generally tubular structure having an upper portion which is widened relative to the tubular body and which is to contain the sample red corpuscles or reagents, a ball being fitted freely in the region where the widened upper portion is separated from the lower portion of the tube, so that, in the narrowed portion in which the ball sits, the microtube has small grooves which define communicating channels between the upper chamber and the lower tube. By selecting the dimensions, it is possible to limit the free sections of the grooves in order to prevent the test sample contained in the upper portion of the tube from passing towards the lower portion which contains the agglutination reagents except by a centrifugation action having predetermined characteristics. Thus, uncontrolled reactions which could give rise to false readings are prevented.

For a better understanding of the invention, some drawings of a device produced in accordance with the present invention are appended by way of non-limiting example.

Figure 1 is a diagrammatic longitudinal section through the device.

Figure 2 is a sectioned detail, on a larger scale, of the device of Figure 1.

Figure 3 is a plan view of the device of Figure 1.

Figure 4 is a cross-section on the plane indicated in Figure 2.

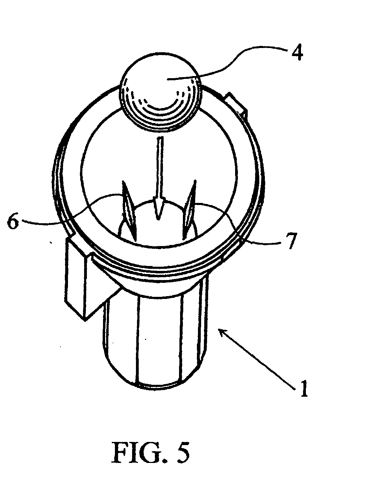

Figure 5 is a perspective view of the situation in respect of a ball for the intermediate separation of the device to which the present invention relates.

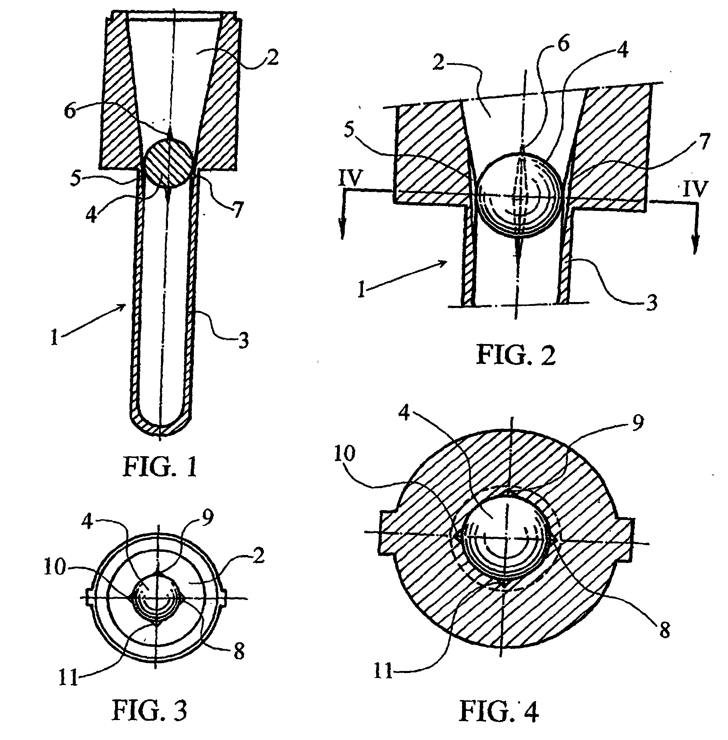

As shown in the Figures, the device to which the present invention relates comprises an element having a generally tubular structure 1, the upper portion 2 of which has dimensions somewhat larger than the properly tubular lower portion 3. The lower portion 3 is to contain the reagents for testing the agglutination of the test sample which will be contained in the upper portion 2 until centrifugation takes place. In order to ensure that the test sample arranged in region cannot pass freely to region 3 in which the agglutination reagents have been placed, before the centrifugation stage, the present invention provides for a ball 4 arranged freely in the intermediate region between the upper cavity 2 and the lower tubular element 3. The transition region has a plurality of grooves such as those indicated by the numerals 5, 6 and 7 in Figure 2, so that, when the ball 4 sits in the transition region, a plurality of small channels, such as those indicated by the numerals 8, 9, 10 and 11, are defined which may be of a size such that, the test liquid contained in the upper widened portion 2 is prevented by the surface tension effect from passing through directly unless a strong dynamic action is exerted on the test sample, such as, for example, a centrifugal action of predetermined value.

Preferably, the grooves are in the form of straight grooves which extend from the lower portion of the upper region of the device as far as the upper portion of the lower region thereof which is to contain the agglutination reagents.

By means of the features described, the device to which the present invention relates cannot permit the passage of the test sample unless it is subjected to the centrifugal action provided for, thus eliminating manual manipulation.

The tubular element 1 may be integrated with other, identical, tubular elements, forming a single support which can be integrated in automatic analysis machines, forming, for example, a "card" or plate carrying a plurality of microtubes 1.

As will be appreciated, the number of grooves and their dimensions are variable, as are the diameters of the ball and of the tubular element so that the passages for liquid can have the desired dimensions.

## Claims

1. A device for carrying out agglutination tests of the type which has a microtube (1) with a widened upper region (2) and a lower tubular region of smaller diameter (3), the purpose of the latter being to contain the agglutination reagents, and the upper region, which has larger dimensions, receiving the test sample, **characterised in that** the device has, in the transition region between the upper portion which is intended for the test sample and the lower portion of the microtube, which lower portion carries the agglutination reagents, a plurality of grooves (5,6,7) on which coincides a freely fitted ball (4), defining, together with the grooves, communication passages (8,9,10,11) between the upper portion and the lower portion of the device which prevent the free passage of the test sample, which can pass to the region carrying the agglutination reagents only if it is subjected to centrifugation having predetermined characteristics.

2. A device for carrying out agglutination tests according to claim 1, **characterised in that** the grooves are in the form of straight grooves which extend from the lower portion of the upper region of the device as far as the upper portion of the lower region thereof which is to contain the agglutination reagents.

3. A device for carrying out agglutination tests, **characterised by** the integration of multiple microtubes according to claim 1, said device thereby forming a unique microtube supporting plate.

## Patentansprüche

1. Vorrichtung zum Durchführen von Agglutinierungstests vom Bauarttyp mit Mikroröhrchen (1) mit einer aufgeweiteten oberen Zone (2) und einer unteren röhrchenförmigen Zone kleineren Durchmessers (3), wobei der Zweck von letzterer darin besteht, Agglutinierungs-Reagenzien aufzunehmen, und die obere Zone, die größere Abmessungen besitzt, die Testprobe aufnimmt, **dadurch gekennzeichnet, daß** die Vorrichtung in der Übergangszone zwischen dem oberen Teil, der für die Testprobe vorgesehen ist, und dem unteren Teil des Mikroröhrchens, der die Agglutinierungs-Reagenzien trägt, eine Mehrzahl von Nuten (5, 6, 7) vorgesehen ist, an denen eine frei eingepaßte Kugel (4) angeordnet ist, die zusammen mit den Nuten Verbindungskanäle (8, 9, 10, 11) zwischen dem oberen Teil und dem unteren Teil der Vorrichtung bildet, die den freien Durchgang der Testprobe verhindert, die zu der die Agglutinierungs-Reagenzien tragenden Zone nur dann gelangen kann, wenn sie einer Zentrifugierung mit vorbestimmten Kennwerten unterzogen wird.

2. Vorrichtung nach Anspruch 1, **dadurch gekennzeichnet, daß** die Nuten die Form von geraden Nuten haben, die sich von dem unteren Teil zu dem oberen Teil der Vorrichtung bis zum oberen Teil der unteren Zone, der die Agglutinierungs―Reagenzien aufzunehmen hat, erstrecken.

3. Vorrichtung zum Durchführen von Agglutinierungstests, **gekennzeichnet durch** die Integration mehrerer Mikroröhrchen nach Anspruch 1, wodurch die Vorrichtung eine einzige Mikroröhrchen-Trägerplatte bildet.

## Revendications

1. Dispositif pour l'exécution d'essais d'agglutination du type qui présente un microtube (1) avec une région supérieure élargie (2) et une région tubulaire inférieure de plus petit diamètre (3), le but de cette dernière étant de contenir les réactifs d'agglutination, et la région supérieure qui présente de plus grandes dimensions, recevant l'échantillon pour essai, **caractérisé en ce que** le dispositif présente, dans la région de transition entre la partie supérieure qui est destinée à l'échantillon pour essai et la partie inférieure du microtube, laquelle partie inférieure porte les réactifs d'agglutination, une pluralité de rainures (5, 6, 7) auxquelles correspond une bille librement ajustée (4), définissant avec les rainures, des passages de communication (8, 9, 10, 11) entre la partie supérieure et la partie inférieure du dispositif qui interdisent le libre passage de l'échantillon pour essai, qui ne peut rejoindre la région portant les réactifs d'agglutination que s'il est soumis à une centrifugation ayant des caractéristiques prédéterminées.

2. Dispositif pour l'exécution d'essais d'agglutination selon la revendication 1, **caractérisé en ce que** les rainures ont la forme de rainures droites qui s'étendent de la partie inférieure de la région supérieure du dispositif jusqu'à la partie supérieure de sa région inférieure qui doit contenir les réactifs d'agglutination.

3. Dispositif pour l'exécution d'essais d'agglutination, **caractérisé par** l'intégration de microtubes multiples selon la revendication 1, ledit dispositif formant une plaque unique de support des microtubes.
